# EUROPEAN PATENT SPECIFICATION

(11) **EP 2 731 595 B1**
(45) Date of publication and mention of the grant of the patent: **04.09.2019**
(21) Application number: 12810730.7
(22) Date of filing: 30.04.2012
(51) Int. Cl.: A61K 31/047, A61P 27/02

(54) **USE OF XANTHOPHYLL CAROTENOIDS TO IMPROVE VISUAL PERFORMANCE AND NEURAL EFFICIENCY**
VERWENDUNG VON XANTHOPHYLL-CAROTENOIDEN ZUR VERBESSERUNG DER SEHFÄHIGKEIT UND DER NERVENEFFIZIENZ
UTILISATION DE CAROTÉNOÏDES XANTHOPHYLLES POUR AMÉLIORER LA PERFORMANCE VISUELLE ET L'EFFICACITÉ NEURALE

(30) Priority: 13.07.2011 US 201161507451 P
(43) Date of publication of application: 21.05.2014
(73) Proprietor: University Of Georgia Research Foundation, Inc., Athens, GA 30602-7411 (US)
(72) Inventor: RENZI, Lisa, M., Watkinsville, GA 30677 (US); HAMMOND, Billy, R., Watkinsville, GA 30677 (US)
(74) Representative: Epping - Hermann - Fischer
(86) International application number: PCT/US2012/035766
(87) International publication number: WO 2013/009378

(56) References cited:
- WO-A1-2009/129859
- WO-A1-2010/125516
- WO-A2-2007/029008
- WO-A2-2007/046083
- GB-A- 2 301 775
- US-A- 5 382 714
- US-A1- 2006 088 574
- US-A1- 2007 082 066
- US-A1- 2008 070 980
- US-A1- 2009 118 228
- US-A1- 2009 264 681
- US-A1- 2009 311 761
- US-A1- 2011 144 200
- US-B2- 6 329 432
- JAMES M. STRINGHAM ET AL: "The Influence of Dietary Lutein and Zeaxanthin on Visual Performance", JOURNAL OF FOOD SCIENCE, vol. 75, no. 1, 1 January 2010 (2010-01-01), pages R24-R29, XP055171405, US ISSN: 0022-1147, DOI: 10.1111/j.1750-3841.2009.01447.x
- JAMES M STRINGHAM ET AL: "FEATURE ARTICLE ON LINE Macular Pigment and Visual Performance Under Glare Conditions", OPTOMETRY AND VISION SCIENCE., vol. 85, no. 2, 1 February 2008 (2008-02-01), pages 82-88, XP055171390, US ISSN: 1040-5488
- LISA M. RENZI ET AL: "The relation between the macular carotenoids, lutein and zeaxanthin, and temporal vision", OPHTHALMIC AND PHYSIOLOGICAL OPTICS., vol. 30, no. 4, 12 July 2010 (2010-07-12), pages 351-357, XP055171993, GB ISSN: 0275-5408, DOI: 10.1111/j.1475-1313.2010.00720.x
- D. MAX SNODDERLY.: 'Evidence for protection against age-related macular degeneration by carotenoids and antioxidant vitamins.' AM. J. CLIN. NUTR. vol. 62, 1995, pages 1448S - 1461S, XP000603641
- KHACHIK, FREDERICK ET AL.: 'Isolation and Structural Elucidation of (13Z,13'Z, 3R,3'R,6'R)-Lutein from Marigold Flowers, Kale, and Human Plasma.' J. AGRIC. FOOD CHEM. vol. 47, 1999, pages 455 - 461, XP055142700
- BOVIER EMILY R ET AL: "Macular Pigment: Relations To Fixed And Variable Reaction Time And Coincidence Anticipation Across The Lifespan", INVESTIGATIVE OPHTHALMOLOGY & VISUAL SCIENCE - IOVS, ASSOCIATION FOR RESEARCH IN VISION AND OPHTHALMOLOGY, US, vol. 52, 1 April 2011 (2011-04-01), page 3621, XP009193604, ISSN: 0146-0404

## Description

### Related Application

This application claims priority from U.S. Provisional Application No. 61/507,451, filed July 13, 2011 and entitled "Use of Lutein and Zeaxanthin to Improve Visual Performance and Neural Efficiency".

### Field of the Invention

The invention provides a non-therapeutic use of Zeaxanthin (Z) in a method of improving reaction time and coincidence anticipation ability in a subject in need.

### Background of the Invention

Xanthophyll carotenoids lutein (L) and zeaxanthin (Z) are found in high concentration in human nervous tissue, such as retina and neocortex [1, 2]. In the retina, L and Z are located in the macula and are termed macular pigment (MP). Thus, MP is composed of the carotenoids lutein (L) and zeaxanthin (Z) and a product of their interconversion, meso-zeaxanthin (MZ). MP optical density can be measured non-invasively using established psychophysical techniques[3], and MP optical density relates strongly to L and Z concentrations in the brain. MP is thought to protect the retina from actinic damage and oxidative stress, and to improve visual function by two basic mechanisms: short-wave light absorption, and improving neural efficiency.

While high MP density may be beneficial for the population at large, athletes who play outdoor sports may receive extra benefit from maintaining high MP optical density (MPOD), as they are required to perform a number of visual and visual-motor tasks at extremely high speed in the very environmental and lighting conditions known to degrade visual function the most.

L and Z function in the retina is relatively well-understood. The following three basic hypotheses have been posed in past literature to explain what L and Z as MP may do to improve visual function. The first hypothesis is the protective hypothesis, which suggests that because L and Z are pigments that both absorb damaging short-wave "blue" light and serve as antioxidants, MP may be able to improve visual function by preventing acquired ocular diseases that degrade vision, such as age-related macular degeneration (AMD), the leading cause of blindness in the West.

The second hypothesis for MP function is based on optical properties of MP. Because L and Z absorb short-wave light, and because short-wave light scatters readily in the atmosphere and within the eyes, MP's ability to absorb short-wave light improves visual performance in short-wave dominant viewing conditions, and in those conditions where absorbing the short-wave portion of a relatively intense broad band light source improves vision. For example, individuals with higher MPOD tend to have improved visual function under glare conditions and in the presence of light stressors. Individuals with higher MP also have improved ability to detect the edge of an object when that object is presented in front of a short-wave background, such as a "blue" sky.

The third hypothesis for MP function is the neural efficiency hypothesis. The neural efficiency hypothesis suggests that MP, on the level of the neural retina and as a biomarker of cortical L and Z concentration, is capable of improving neural efficiency by reducing neural noise (random neural firing that is not correlated to the presence of a sensory stimulus), by improving processing speed, and by minimizing the amount of cortical area necessary to perform for any given cognitive task.

A relatively large body of literature has supported the first two hypotheses (the protective hypothesis and the optical hypotheses). Renzi and Hammond [4] posited the neural efficiency hypothesis in 2010 and have been collecting data to determine whether or not the neural efficiency hypothesis is correct.

Notwithstanding the aforementioned efforts to understand the role of cortical L and Z in optic health, the need continues to exist for treatments and pharmaceutical compositions that can utilize Xanthophyll carotenoids to improve visual performance and neural efficiency.

Stringham J. et al., Journal of Food Science, vol. 75, pages R24-R29 teaches positive effects of lutein and zeaxanthin on photostress recovery.

Stringham et al., Optometry and Vision Science, vol 85, no. 2, pp. 82-88 discloses the administration of compounds including lutein and zeaxanthin to relate macular pigment to improvements in glare disability and photostress recovery.

WO 2010/125516 A1 discloses compositions containing a mixture of lutein and zeaxanthin for use in the treatment, human or veterinarian, of diseases or disorders of the eye.

US 2007/082066 A1 discloses the use of zeaxanthin for reducing light hyper-sensitivity,photophobia and medical conditions relating to light hyper-sensitivity.

US 2009/118228 A1 discloses administering a combination of lutein, zeaxanthin, lycopene and beta-carotene for (1) improving optical health, (2) preventing photoreceptor cell death, (3) protecting eyes from light and sun damage, (4) improving skin health, and (5) preventing obesity.

WO 2009/129859 A1 discloses compositions for visual acuity loss in subjects having, or at risk of developing, ocular diseases, such as macular degeneration, diabetic retinopathy, and others.

GB 2 301775 A discloses the use of lutein and zeaxanthin for treating age-related macular degeneration.

US 2006/088574 A1 discloses administering nutritional supplements to individuals for balanced nutrition, for maintaining blood glucose levels, and for those at risk of developing glucose intolerances or cardiovascular diseases, including diabetic, pregnant and/or lactating, and geriatric subjects.

Bovier E. et al., Investigative Ophthalmology & Visual Science - IOVS, vol. 52, p. 3621 discloses a study that investigated whether high macular pigment optical density relates to functional changes such as improved reaction time and coincidence anticipation by studying "individuals across the lifespan."

### Summary of the Invention

The present invention provides a non-therapeutic use of Zeaxanthin (Z) in a method of improving reaction time and coincidence anticipation ability in a subject in need according to claim 1. Preferred embodiments are set forth in the subclaims.

Preferably, a pharmaceutically effective amount of Zeaxanthin (Z) is administered topically to the subject (or by the subject to himself or herself) by application of an ocular solution, or is administered systemically through a solid or liquid dosage form comprising not less than about 200%, or not less than about 190%, or not less than about 180%, or not less than about 170%, or not less than about 160%, or not less than about 150%, or not less than about 140%, or not less than about 130%, or not less than about 120%, or not less than about 110% of the amount of Zeaxanthin (Z) that might otherwise be metabolized by a subject as a result of eating food sources such as eggs, spinach, or corn. For example, using purely illustrative ranges, pharmaceutically effective amounts of zeaxanthin (Z) could range from about 1,000 mg, or about 900 mg, or about 800 mg, or about 700 mg, or about 600 mg, or about 500 mg, or about 400 mg, or about 300 mg, or about 200 mg, or about 100 mg, or about 900 mg, or about 90 mg, or about 80 mg, or about 70 mg, or about 60 mg, or about 50 mg, or about 40 mg, or about 30 mg, or about 20 mg, or about 10 mg, or about 9 mg, or about 8 mg, or about 7 mg, or about 6 mg, or about 5 mg, or about 4 mg, or about 3 mg, or about 2 mg, or about 1 mg. In certain aspects of the invention, the composition is in the form of a food composition, including a food bar such as a sports bar or a liquid.

### Brief Description of the Figures

**Figure 1****.** Stimuli and timing characteristics for CAT, FRT and VRT as determined for healthy subjects in the experiment of Example 1.
**Figure 2****.** Improvements for one subject, baseline MPOD of 0.7 at 30-min of eccentricity, 12-week MPOD of 0.77, as determined for healthy subjects in the experiment of Example 1.

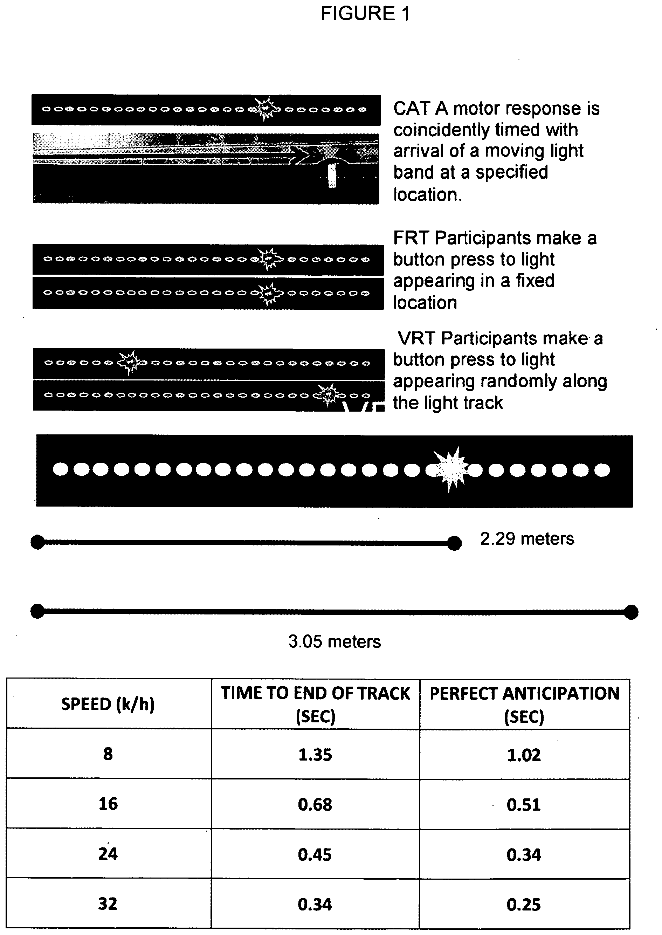

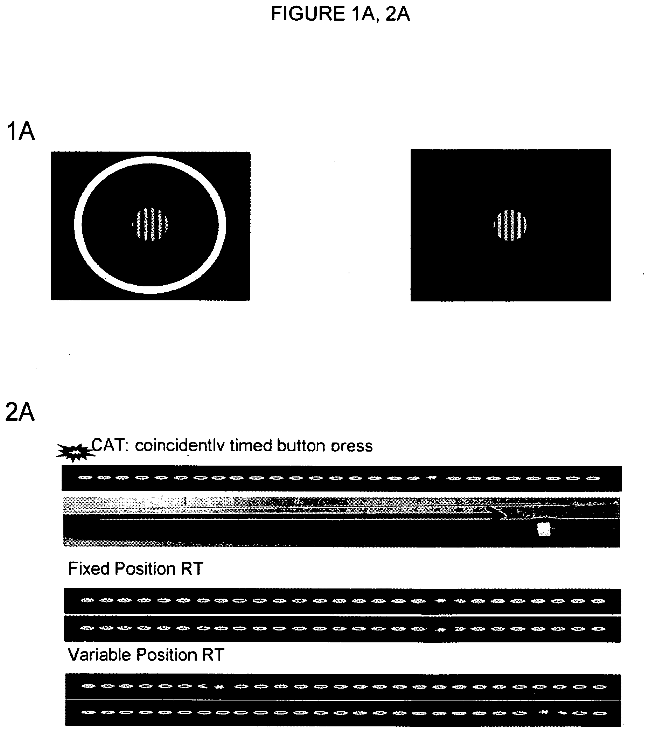

**Figure 1A****.** Glare disability testing results determined in the experiment of Example 2; stimuli presented in three-channel Maxwellian-view optical system (1 kW xenon-arc light source): (a) 570nm target (100% contrast, 5 cycles per degree - cpd); 10° inner, 12° outer diameter annulus comprised of broad-band xenon light. Contrast enhancement testing results determined in the experiment of Example 2: (b) 600nm target (100% contrast grating, 5 cpd); 460nm surround.

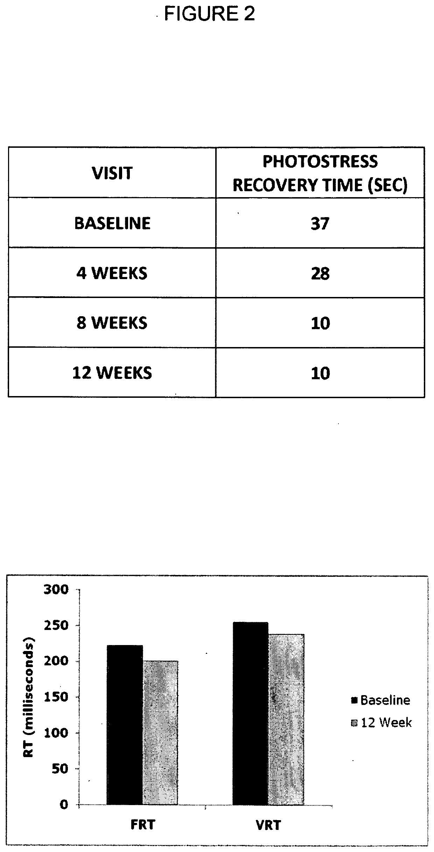

**Figure 2A****.** Dynamic visual performance results determined in the experiment of Example 2.

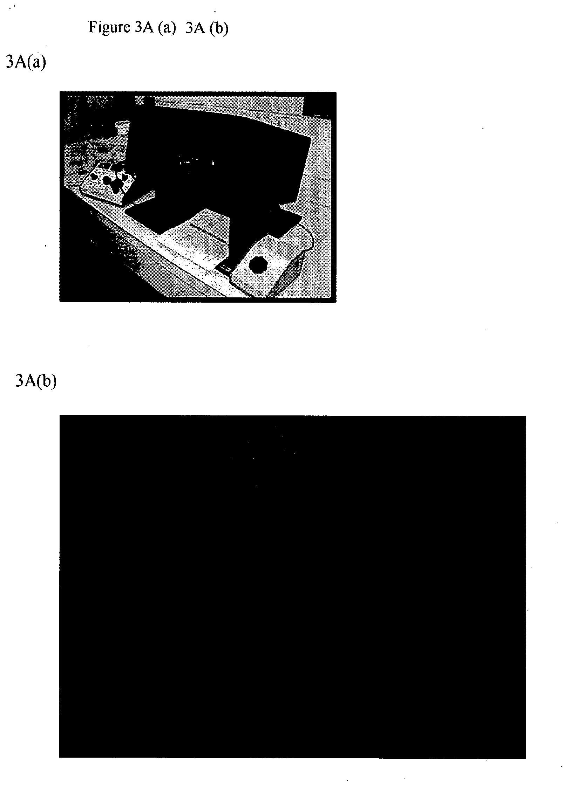

**Figure 3A**. Temporal Contrast Sensitivity Function (TCSF) apparatus (a) and Free-view presentation (through an artificial pupil; see Figure 3a) of 1°, 660nm target centered within 10°, 660nm surround (b); results determined in the experiment of Example 2.

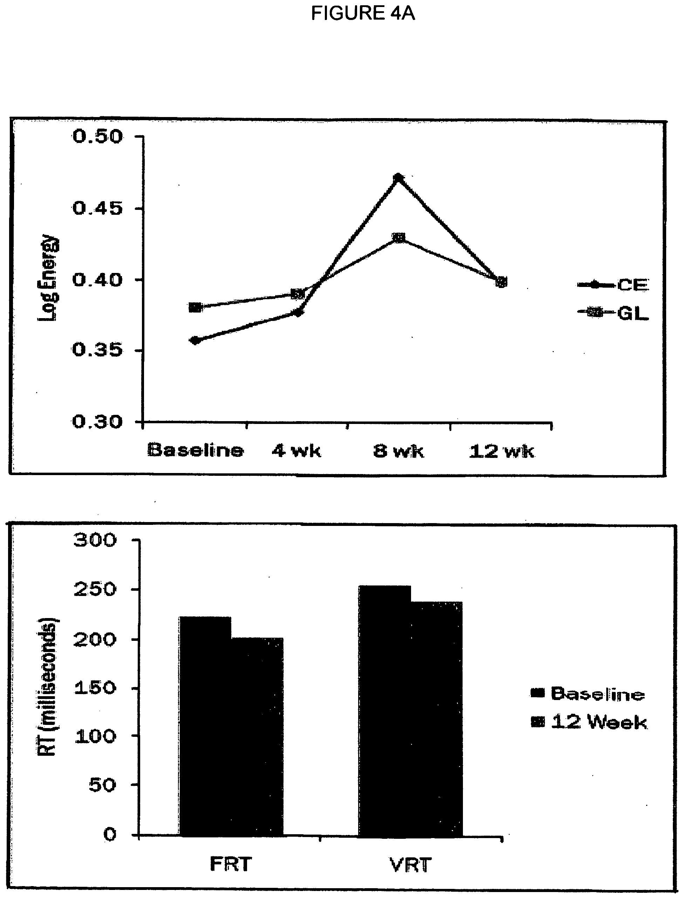

**Figure 4A****.** Increases in MPOD and improvement in both static and dynamic visual performance after zeaxanthin supplementation results determined in the experiment of Example 2.

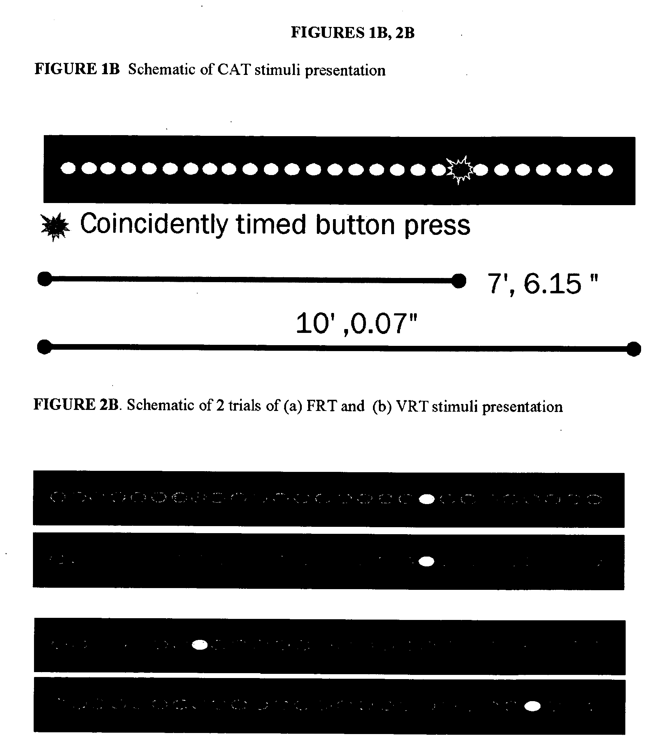

**Figure 1B**. Schematic of CAT stimuli presentation; results determined in the experiment of Example 3.
**Figure 2B****.** Fixed and variable position reaction time; results determined in the experiment of Example 3.

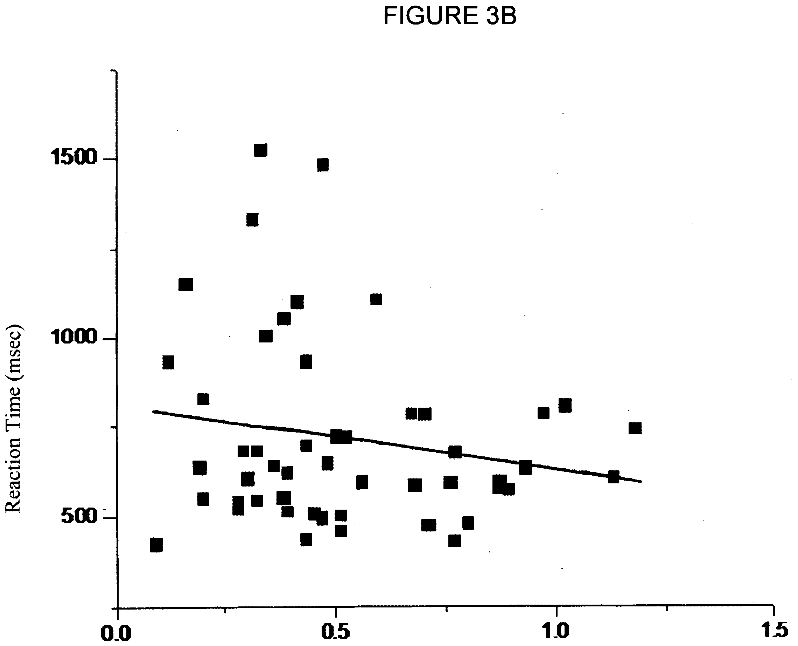

**Figure 3B****.** MPOD was significantly related to decreased RT (*r* = *-*0.25, *p* < 0.05); results determined in the experiment of Example 3.

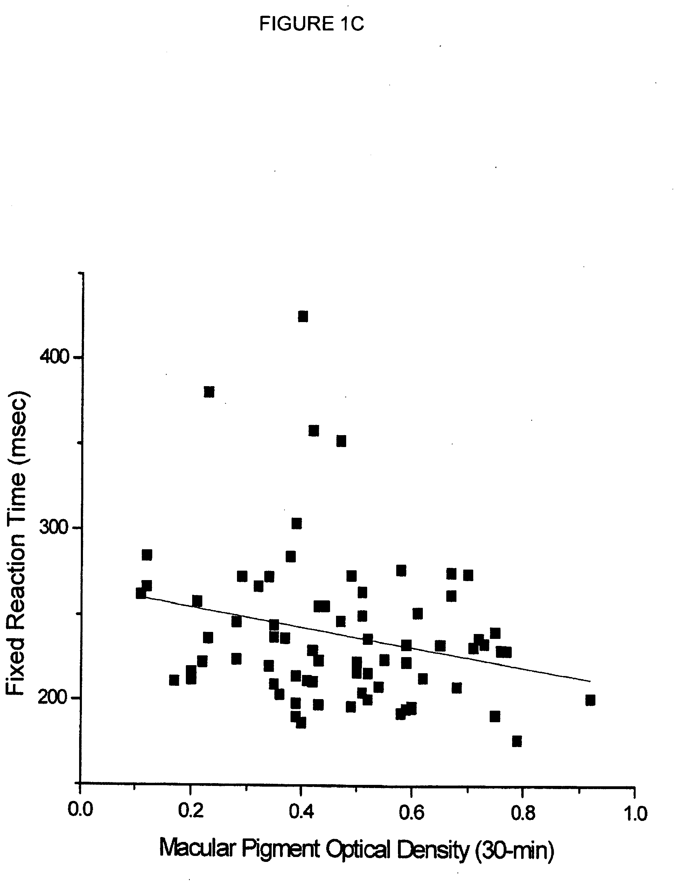

**Figure 1C****.** The relationship between MPOD and fixed position reaction time in young, healthy adults. (*r* = -0.21, *p* < 0.05); results determined using a protocol similar to that described in the experiment of Example 4.

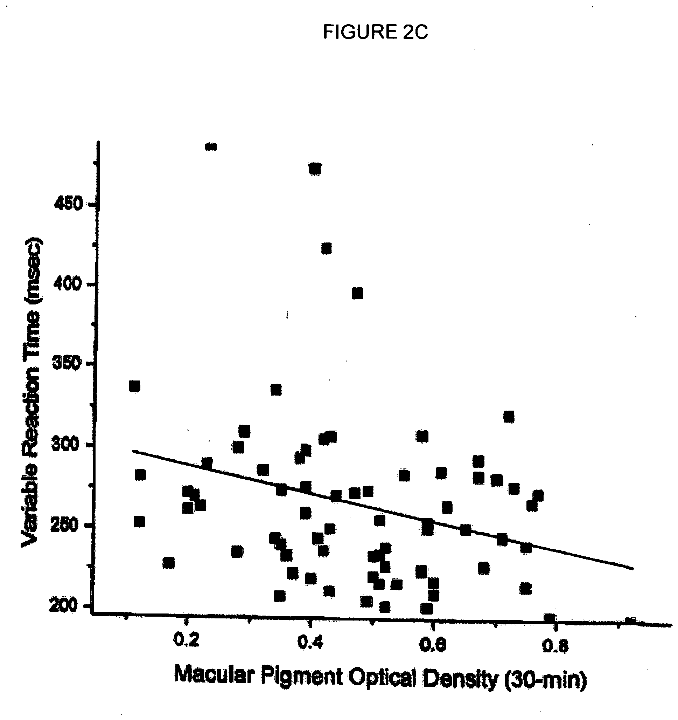

**Figure 2C****.** The relationship between MPOD and variable position reaction time in young, healthy adults. (*r* = -0.22, *p* < 0.05); results determined using a protocol similar to that described in the experiment of Example 4.

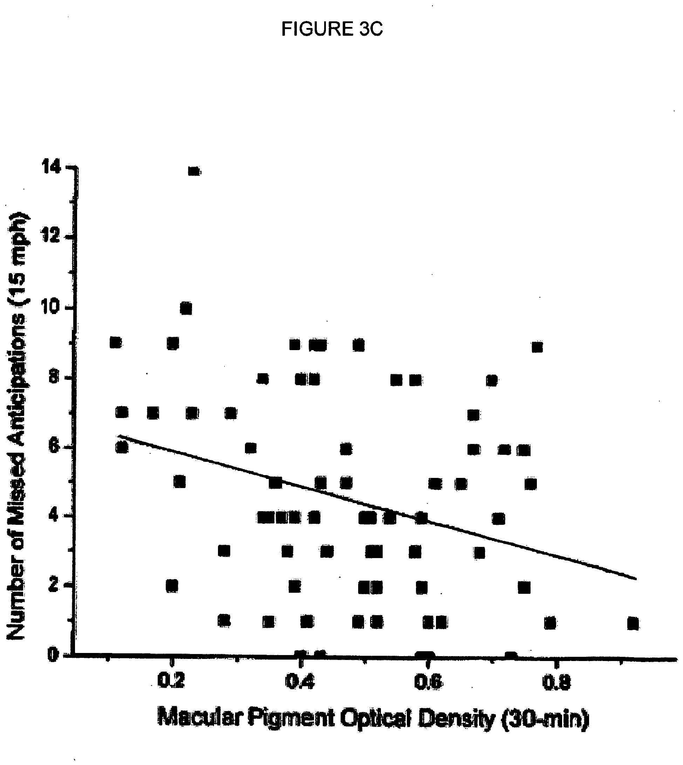

**Figure 3C****.** The relationship between error in coincidence anticipation and MPOD in young, healthy adults. (*r* = -0.23, *p* < 0.05); results determined using a protocol similar to that described in the experiment of Example 4.

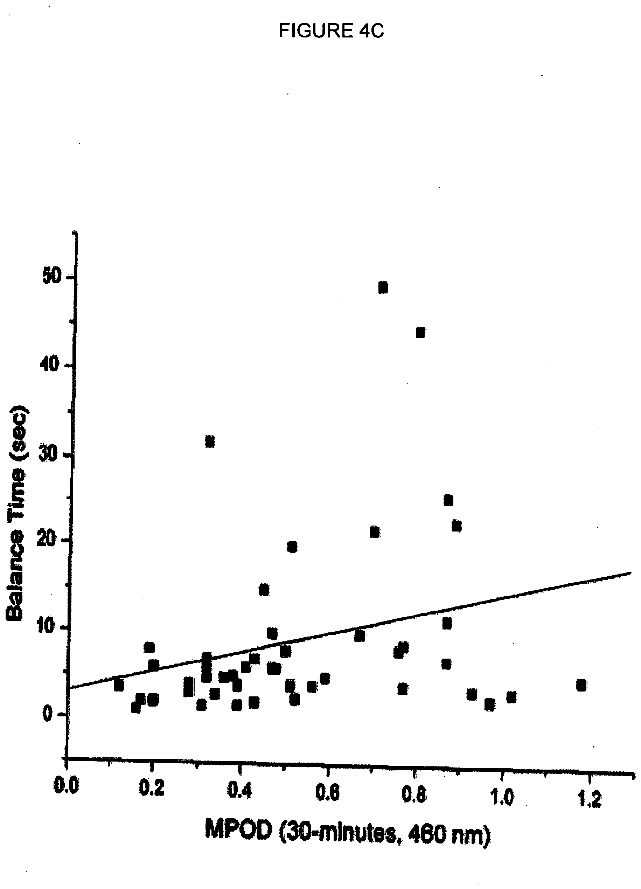

**Figure 4C**. The relationship between MPOD and balance time in healthy elders. (*r* = 0.29, *p* < 0.05); results determined using a protocol similar to that described in the experiment of Example 4.

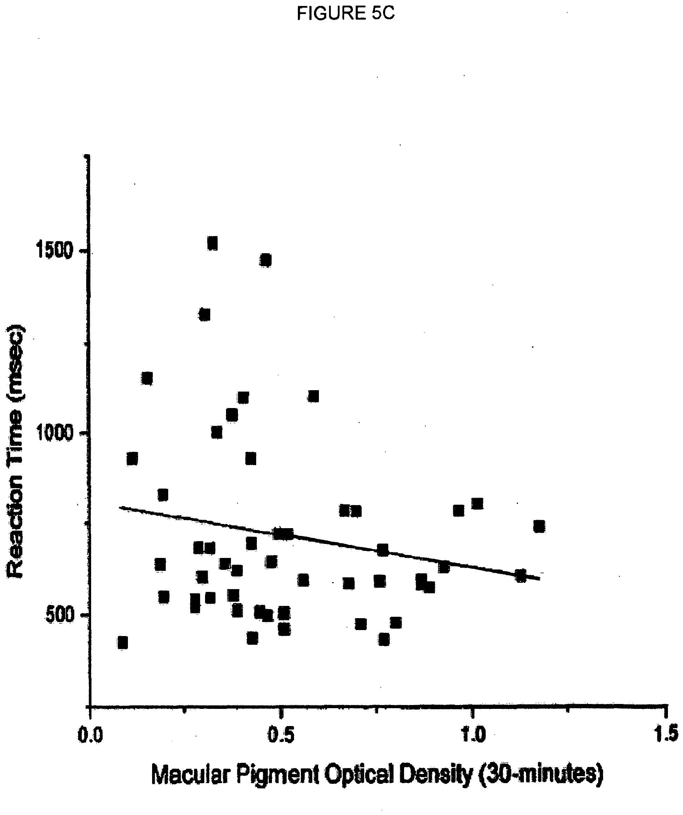

**Figure 5C****.** The relationship between MPOD and judgment reaction time in healthy elders. (*r* = -0.25, *p* < 0.05); results determined using a protocol similar to that described in the experiment of Example 4.

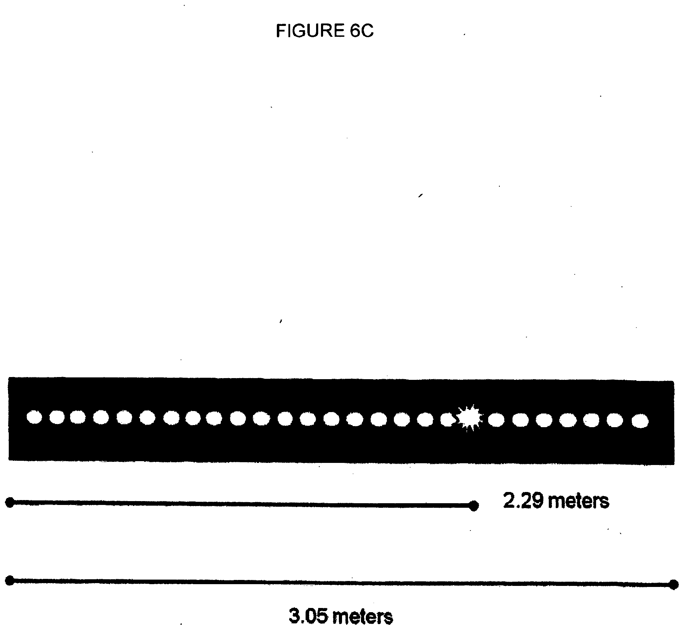

**Figure 6C****.** Schematic of the linear light array used to determine FRT, VRT and CAT in accordance with the experiment described in Example 4.

### Detailed Description of the Invention

The following terms, among others, are used to describe the present invention. It is to be understood that a term which is not specifically defined is to be given a meaning consistent with the use of that term within the context of the present invention as understood by those of ordinary skill.

It is noted that, as used in this specification and the appended claims, the singular forms "a," "an," and "the," include plural referents unless expressly and unequivocally limited to one referent. Thus, for example, reference to "a compound" includes two or more different compound. As used herein, the term "include" and its grammatical variants are intended to be non-limiting, such that recitation of items in a list is not to the exclusion of other like items that can be substituted or other items that can be added to the listed items.

"Improved reaction time and coincidence anticipation ability" and "enhanced static and dynamic visual performance" can be assessed in a variety of ways, e.g. using the methodologies described in Examples 1-4 herein. Relevant parameters indicative of improved reaction time and coincidence anticipation ability include but are not limited to:
(1) an increase in MPOD of about 100%, 95%, 90%, 85%, 80%, 75%, 70%, 65%, 60%, 55%, 50%, 45%, 40%, 35%, 30%, 25%, 20%, 15%, 10%, or 5% compared to a subject's baseline MOPD level;
(2) an increase in MPOD associated with a decrease in photostress recovery time of about 10, or 9 or 8 or 7 or 6 or 5 or 4 or 3 or 2 or 1 seconds (e.g. decreased photostress recovery time associated with a MPOD increase of around 10% to around 15% of a baseline MPOD level);
(3) decreases in CAT, FRT, and/or VRT of about 100%, 95%, 90%, 85%, 80%, 75%, 70%, 65%, 60%, 55%, 50%, 45%, 40%, 35%, 30%, 25%, 20%, 15%, 10%, or 5% compared to a subject's baseline CAT, FRT, and/or VRT levels;
(4) a critical flicker fusion threshold increase of between around 0.1, 0.2, 0.3, 0.4, 0.5, 0.6, 0.7, 0.8, 0.9, 1.0, 1.1, 1.2, 1.4, 1.4, 1.5, 1.6, 1.7, 1.8, 1.9, 2.0, 2.1, 2.2, 2.3, 2.4, 2.5, 2.6, 2.7, 2.8, 2.8, 2.9, or 3.0 Hz; and
(4) a logarithmic increase in disability glare threshold (veiled glare threshold)) of around 0.01 to 0.25 over a period of around one to around six months;

"Lighting conditions that are known to be detrimental to visual function" include but are not limited to lighting conditions in which blue light (e.g. "blue haze") is a major factor in limiting outdoor vision (e.g. light at wavelengths of around 400 nm to around 550 nm), and disability glare conditions associated with xenon-white light at log energy (µW/cm²) values of around 2 to around 4.

"Increases in MPOD" can be measured over a wide variety of time points, including but not limited to one or more days, weeks , or months (e.g. around a 50% increase in MPOD over a period of around 120 days).

"Preventing continued visual acuity deterioration in a subject who suffers from age-related macular degeneration" includes but is not limited to improving standardized visual acuity, optical coherence tomography (OCT), macular thickness and volume, and intraocular pressure, decreasing central foveal thickness from around 400 to around 300 or around 250 microns as measured by OCT, fluorescein angiography and OCT demonstrated cessation of vascular leakage, resolution of hemorrhage and subretinal fluid in the treated eye, improved scores on the National Eye Institute Vision Function Questionnaire (NEI VFQ), the Activities Inventory (AI), and the Veterans Affairs Low Vision Visual Functioning Questionnaire (VA LV VFQ-48) or Targeted vision Test, or in a sample protocol achieving the following results: test corrected visual acuity improved from hand motions to 20/800 (and improved from 0 to 5 letters on the Early Treatment Diabetic Retinopathy Study [ETDRS] visual acuity chart) in the study eye of a patient with Stargardt's macular dystrophy, and vision also improves in a patient with dry age-related macular degeneration (from 21 ETDRS letters to 28).

"Preventing visual acuity deterioration in a subject who is at risk of developing age-related macular degeneration" can entail preventing increases in macular thickness and volume in a subject who is around fifty years of age of older, as well as decreasing such a subject's intraocular pressure and central foveal thickness, and preventing an accumulation in the subject's eye of sub-RPE deposits that contain molecular constituents of human drusen, decreased segmentation of atrophic areas in the subject's eye as confirmed by Fundus autofluorescence imaging, decreased GA enlargement in the subject's eye, and confirmation of a lack distinct microstructural alterations related to GA as visualized using high-resolution spectral-domain optical coherence tomography.

"Xanthophyll carotenoids" generally refers to a naturally occurring or synthetic 40-carbon polyene chain with a carotenoid structure that contains at least one oxygen-containing functional group. The chain may include terminal cyclic end groups. Exemplary, though non-limiting, xanthophyll carotenoids include astaxanthin, zeaxanthin, lutein, echinenone, lycophyll, canthaxanthin, and the like. Isomerism around carbon-carbon double bonds yields distinctly different molecular structures that may be isolated as separate compounds (known as Z ("cis") and E ("trans"), or geometric, isomers). Xanthophyll carotenoids therefore include but are not limited to (3R, 3'R, 6'R)-lutein, (3R, 3'R, 6'R)-zeaxanthin, the (E / Z) isomers of (3R, 3'R, 6'R)-lutein and (3R, 3'R; 6'R)-zeaxanthin, the metabolites (3R,3'S,6'R)-lutein (3'-epilutein) and 3-hydroxy-β,ε -caroten-3'-one, (3R,3'S-meso)-zeaxanthin (meso-zeaxanthin (MZ)), 3'-oxolutein, 3-methoxyzeaxanthin (3-MZ), β-cryptoxanthin, epsilon-lycopenes, 5-Z-lycopenes, and apo-carotenoid products including 3-OH-β-ionone, 3-OH-α-ionone, β**-**ionone, 3-OH-α-apo-10'-carotenal, 3-OH-β-apo-10'-carotenal, and β-apo-10'-carotenal.

"Substantially pure enantiomeric form" as used herein comprises greater than about 80% by weight of a particular enantiomeric form of xanthophyll carotenoid (e.g. (3R, 3'R, 6'R)-zeaxanthin) and less than about 20% by weight of another enantiomeric form of that xanthophyll carotenoid, more preferably greater than about 90% by weight of the particular enantiomeric form of the xanthophyll carotenoid and less than about 10% by weight of another enantiomeric form of that xanthophyll carotenoid, even more preferably greater than about 95% by weight of the particular enantiomeric form xanthophyll carotenoid and less than about 5% by weight of another enantiomeric form of that xanthophyll carotenoid, and most preferably greater than about 99% by weight of particular enantiomeric form of xanthophyll carotenoid and less than about 1% by weight of another enantiomeric form of that xanthophyll carotenoid. A "substantially pure xanthophyll carotenoid derivative" is defined similarly with respect to the relative amounts of its enantiomers.

The term "compound", as used herein, unless otherwise indicated, refers to any specific chemical compound disclosed herein and includes tautomers, regioisomers, geometric isomers, and where applicable, optical isomers (e.g. enantiomers) thereof, as well as pharmaceutically acceptable salts and derivatives (including prodrug forms) thereof. Within its use in context, the term compound generally refers to a single compound, but also may include other compounds such as stereoisomers, regioisomers and/or optical isomers (including racemic mixtures) as well as specific enantiomers or enantiomerically enriched mixtures of disclosed compounds as well as diastereomers and epimers, where applicable in context. The term also refers, in context to prodrug forms of compounds which have been modified to facilitate the administration and delivery of compounds to a site of activity.

"Fatty acids" include but are not limited to essential fatty acids, omega-3, omega-6, and omega-9 fatty acids, and trans fatty acids.

The term "patient" or "subject" is used throughout the specification within context to describe an animal, generally a mammal and preferably a human, to whom treatment, including prophylactic treatment (prophylaxis), with the compositions according to the present invention is provided. For treatment of those infections, conditions or disease states which are specific for a specific animal such as a human patient, the term patient refers to that specific animal.

The term "effective" is used herein, unless otherwise indicated, to describe an amount of a compound or composition which, in context, is used to produce or effect an intended result, whether that result relates to the enhancement of a subject's macular pigment optical density, improving the static and dynamic visual performance of a subject, improving reaction time and coincidence anticipation ability in a subject who is required to perceive and react to temporally varying stimuli under lighting conditions that are known to be detrimental to visual function. This term subsumes all other effective amount or effective concentration terms (including the term "therapeutically effective") which are otherwise described in the present application.

The terms "treat", "treating", and "treatment", etc., as used herein, include improving the static and dynamic visual performance of a subject, improving reaction time and coincidence anticipation ability in a subject who is required to perceive and react to temporally varying stimuli under lighting conditions that are known to be detrimental to visual function. Treatment, as used herein, encompasses both prophylactic and therapeutic treatment and also includes self-treatment (e.g. a subject without the assistance of any intermediary ingests or applies a Xanthophyll carotenoid to himself or herself).

The term "pharmaceutically acceptable salt" or "salt" is used throughout the specification to describe a salt form of one or more of the compositions herein which are presented to increase the solubility of the compound in saline, most preferably in order to promote dissolution and the bioavailability of topically applied or orally ingested compounds. Pharmaceutically acceptable salts include those derived from pharmaceutically acceptable inorganic or organic bases and acids. Suitable salts include those derived from alkali metals such as potassium and sodium, alkaline earth metals such as calcium, magnesium and ammonium salts, among numerous other acids well known in the pharmaceutical art. Sodium and potassium salts may be preferred as neutralization salts of carboxylic acids and free acid phosphate containing compositions according to the present invention. The term "salt" shall mean any salt consistent with the use of the compounds according to the present invention. In the case where the compounds are used in pharmaceutical indications, the term "salt" shall mean a pharmaceutically acceptable salt, consistent with the use of the compounds as pharmaceutical agents.

The term "co-administration" shall mean that at least two compounds or compositions or treatment regimens are administered to the patient at the same time, such that effective amounts or concentrations or effects of each of the two or more compounds or treatment regimens may be found in the patient at a given point in time. Although compounds according to the present invention may be co-administered to a patient at the same time, the term embraces both administration of two or more agents or treatment regimens at the same time or at different times, including sequential administration. Preferably, effective concentrations of all co-administered compounds or compositions or regimens are found in the subject at a given time.

The term "ester", as used herein, refers to a group --C(O)O-substituent wherein the substituent represents, for example, a hydrocarbyl or other substitutent as is otherwise described herein.

Compositions, including pharmaceutical compositions, comprising combinations of an effective amount of Zeaxanthin (Z), and one or more of the compounds otherwise described herein, all in effective amounts, in combination with a pharmaceutically effective amount of a carrier, additive or excipient, are set forth as reference examples.

The reference compositions may be formulated in a conventional manner using one or more pharmaceutically acceptable carriers and may also be administered in controlled-release formulations. Pharmaceutically acceptable carriers that may be used in these pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as prolamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The reference compositions may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally or topically. Oral compositions also may be presented in the form of a food product or liquid drink.

Sterile injectable forms of the reference compositions may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1, 3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as Ph. Helv or similar alcohol.

The reference compositions may be orally administered in any orally acceptable dosage form including, but not limited to, capsules, tablets, aqueous suspensions or solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are required for oral use, the active ingredient is combined with emulsifying and suspending agents. If desired, certain sweetening, flavoring or coloring agents may also be added as well as a food base, especially to provide a food or liquid composition, for example, in the form of a sports bar or sports drink.

Alternatively, the reference compositions may be administered in the form of suppositories for rectal administration. These can be prepared by mixing the agent with a suitable non-irritating excipient which is solid at room temperature but liquid at rectal temperature and therefore will melt in the rectum to release the drug. Such materials include cocoa butter, beeswax and polyethylene glycols.

The reference compositions may also be administered topically. Suitable topical formulations are readily prepared for each of these areas or organs. Topical application for the lower intestinal tract can be effected in a rectal suppository formulation (see above) or in a suitable enema formulation. Topically-acceptable transdermal patches may also be used.

For topical applications, the reference compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water.

Alternatively, the reference compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For topical ophthalmic use, the reference compositions may be formulated as micronized suspensions in isotonic, pH adjusted sterile saline, or, preferably, as solutions in isotonic, pH adjusted sterile saline, either with our without a preservative such as benzylalkonium chloride. Alternatively, for ophthalmic uses, the pharmaceutical compositions may be formulated in an ointment such as petrolatum.

The reference compositions may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The amount of compound in a reference composition that may be combined with the carrier materials to produce a single dosage form will vary depending upon the host and disease treated, the particular mode of administration. Preferably, the reference compositions should be formulated to contain between about 0.05 milligram to about 750 milligrams or more, more preferably about 1 milligram to about 600 milligrams, and even more preferably about 10 milligrams to about 500 milligrams of active ingredient, alone or in combination with at least one additional active ingredient which may be used to improve one or more of the static and dynamic visual performance of a subject and improve reaction time and coincidence anticipation ability in a subject, including a subject who is required to perceive and react to temporally varying stimuli under lighting conditions that are known to be detrimental to visual function.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease or condition being treated.

The active compound is included in the pharmaceutically acceptable carrier or diluent in an amount sufficient to deliver to a patient a therapeutically effective amount for the desired indication, without causing serious toxic effects in the patient treated. A preferred dose of the active compound for all of the herein-mentioned conditions is in the range from about 10 ng/kg to 300 mg/kg, preferably 0.1 to 100 mg/kg per day, more generally 0.5 to about 25 mg per kilogram body weight of the recipient/patient per day. A typical topical dosage will range from 0.01-3% wt/wt in a suitable carrier.

The compound is conveniently administered in any suitable unit dosage form, including but not limited to one containing less than 1mg, 1 mg to 3,000 mg, preferably 5 to 500 mg of active ingredient per unit dosage form. An oral dosage of about 25-250 mg is often convenient.

In a reference example, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art.

Liposomal suspensions may also be pharmaceutically acceptable carriers. These may be prepared according to methods known to those skilled in the art, for example, as described in U.S. Pat. No. 4,522,811. For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound is then introduced into the container. The container is then swirled by hand to free lipid material from the sides of the container and to disperse lipid aggregates, thereby forming the liposomal suspension.

Representative reference compositions include, but are not limited to the following. A reference composition (e.g. a topically-applied ophthalmic composition or an orally ingestable composition, including a food composition or liquid drink) comprising:
(a) about 1,000 mg, or about 900 mg, or about 800 mg, or about 700 mg, or about 600 mg, or about 500 mg, or about 400 mg, or about 300 mg, or about 200 mg, or about 100 mg, or about 900 mg, or about 90 mg, or about 80 mg, or about 70 mg, or about 60 mg, or about 50 mg, or about 40 mg, or about 30 mg, or about 20 mg, or about 10 mg, or about 9 mg, or about 8 mg, or about 7 mg, or about 6 mg, or about 5 mg, or about 4 mg, or about 3 mg, or about 2 mg, or about 1 mg of Zeaxanthin (Z); and optionally
(b) a pharmaceutically acceptable excipient.

In the reference compositions described herein, the Zeaxanthin (Z) can be in substantially pure enantiomeric form.

Another illustrative reference composition comprises:
(a) about 1,000 mg, or about 900 mg, or about 800 mg, or about 700 mg, or about 600 mg, or about 500 mg, or about 400 mg, or about 300 mg, or about 200 mg, or about 100 mg, or about 900 mg, or about 90 mg, or about 80 mg, or about 70 mg, or about 60 mg, or about 50 mg, or about 40 mg, or about 30 mg, or about 20 mg, or about 10 mg, or about 9 mg, or about 8 mg, or about 7 mg, or about 6 mg, or about 5 mg, or about 4 mg, or about 3 mg, or about 2 mg, or about 1 mg of at least Zeaxanthin (Z) selected from the group consisting of (3R, 3'R, 6'R)-zeaxanthin, the (*E* / *Z*) isomers of (3R, 3'R, 6'R)-zeaxanthin, and (3R,3'S-meso)-zeaxanthin (meso-zeaxanthin (MZ)),; and optionally
(b) a pharmaceutically acceptable excipient.

Another illustrative reference composition constitutes an ophthalmic solution or gel comprising at least one active ingredient selected from the group consisting of:
(a) substantially enantiomerically pure (3R, 3'R, 6'R)-lutein, substantially enantiomerically pure (3R, 3'R, 6'R)-zeaxanthin, or a substantially enantiomerically pure (E / Z) isomer of (3R, 3'R, 6'R)-zeaxanthin; and
(b) a pharmaceutically acceptable excipient.

Another illustrative reference composition comprises:
(a) about 1,000 mg, or about 900 mg, or about 800 mg, or about 700 mg, or about 600 mg, or about 500 mg, or about 400 mg, or about 300 mg, or about 200 mg, or about 100 mg, or about 900 mg, or about 90 mg, or about 80 mg, or about 70 mg, or about 60 mg, or about 50 mg, or about 40 mg, or about 30 mg, or about 20 mg, or about 10 mg, or about 9 mg, or about 8 mg, or about 7 mg, or about 6 mg, or about 5 mg, or about 4 mg, or about 3 mg, or about 2 mg, or about 1 mg of Zeaxanthin (Z) selected from the group consisting of (3R,3'S-meso)-zeaxanthin (meso-zeaxanthin (MZ)),; and optionally
(b) a pharmaceutically acceptable excipient.

Another illustrative reference composition constitutes an ophthalmic solution, ointment or gel comprising:
(a) substantially enantiomerically pure (3R,3'S-meso)-zeaxanthin (meso-zeaxanthin (MZ)); and
(b) a pharmaceutically acceptable excipient.

Each of the reference compositions described above may be formulated as a solid or liquid food composition for oral ingestion. Thus, the active compounds described above may be formulated as a sports bar or sports drink and packaged for consumption by the subject, for example, at the site of an athletic event or other location.

In a reference example, a method of treatment comprises administering zeaxanthin to a subject or patient, in particular, an athlete engaging in a sports activity, a driver, including a long distance driver or a night driver, military personnel or aviation personnel, especially including pilots of aircraft in need of enhanced visual performance and/or neural efficiency. In a reference example, the method comprises enhancing visual performance and neural efficiency, thus making the performance of the subject engaging in the activity more effective. It is an unexpected result that effective amounts of zeaxanthin, preferably both lutein and zeaxanthin and optionally fatty acids especially including omega 3 fatty acids, would provide a significant enhance in the visual performance and neural efficiency of the subject. The use of the reference composition to provide a substantially non-toxic means of enhancing visual and neural efficiency (which can include physical performance including hand and eye coordination) is a further reference example.

Notable reference examples include the following:
- Exploiting a link between macular pigment measurement and sports performance;
- Supplementation of zeaxanthin and lutein to build macular pigment;
- Supplementation of zeaxanthin, lutein and Omega-3s to improve sports and other physical performance;
- Measuring sports performance using macular pigment measurement and other visual performance tests;
- Supplement delivery methods including pills, food, drinks, sports bars and powders;
- Specific performance improvements including reaction time, hand and eye coordination, contrast sensitivity and others
- The link between visual performance and sports performance

The use of the reference compositions in a patient or subject results in enhanced visual performance and/or neural efficiency takes the form of one or more of enhanced visual contrast sensitivity, enhanced cognitive performance, enhanced visual efficiency, enhanced motion sensitivity, enhanced spatial memory, enhanced choice reaction, enhanced integration of visual motion (reduction of time), enhanced choice reaction time (reduced), enhanced hand and eye coordination of that individual in the performance of a task or the engagement in an activity for that patient or subject.

The invention is illustrated further in the following non-limiting examples.

### Example 1

### The effects of macular carotenoids lutein and zeaxanthin on visual performance and neural efficiency in young, healthy subjects and college athletes

The purpose of this pilot study was to describe visual performance differences between athletes and other young, healthy adults, and to determine whether or not increasing MP density resulted in performance increases in athletes.

### Methods

### 1. Cross-Sectional Study

Subjects: 78 young, healthy adults (*M*= 20.6 ± 2.6 years), including 16 college baseball players, participated in the cross sectional study. Visual function was measured using MPOD. Neural efficiency was measured as shown in Figure 1. The following additional parameters were also measured: Fixed Reaction Time (FRT); Variable Reaction Time (VRT); and Coincidence Anticipation Timing (CAT).

### Results:

### Cross-Sectional Study:

MPOD did not differ significantly between athletes and non-athletes. FRT did not differ significantly between athletes and non-athletes. VRT was significantly lower in athletes. Athletes were significantly more accurate on CAT than non athletes at high velocities.

### 2. Supplementation Study

Subjects: Seven college baseball players (*M*= 20.3 ±0.58 years), supplemented with 20 mg / Z / day for 3-months, participated in this study. The visual function parameters MPOD, disability glare, photostress recovery time, and contrast enhancement were determined.
The neural efficiency parameters Fixed Reaction Time (FRT); Variable Reaction Time (VRT); and Coincidence Anticipation Timing (CAT) were determined.

### Results:

### Supplementation Study.

Three subjects completed the study. MPOD increased in supplemented subjects (see sample subject, Figure 2). Photostress recovery time decreased in supplemented subjects. Early improvements in glare and contrast enhancement were observed. FRT and VRT improved and
CAT accuracy improved at the highest frequency

### Conclusions

Supplementation to increase MPOD is especially beneficial in athletes who perform outdoors.

### Example 2

### Static and Dynamic Measures of Visual Performance in Athletes

Baseball performance requires the ability to perceive and react to temporally varying stimuli under lighting conditions that are known to be most detrimental to visual function (i.e., short-wave light). Macular pigment (MP) -- lutein (L) and zeaxanthin (Z) in the retina -- is known to improve performance under such conditions and may also improve neural efficiency. The purpose of this study was to assess static and dynamic visual performance in college baseball players in order to:
a) Define performance ability in athletes and non-athletes matched for age and MP (N = 18); and,
b) Improve athletes' performance via supplementation.

### Method: Static Visual Performance

Stimuli presented in three-channel Maxwellian-view optical system (1 kW xenon-arc light source).

Glare Disability (GL)
▪ 570nm target (100% contrast, 5 cycles per degree - cpd); 10° inner, 12° outer diameter annulus comprised of broad-band xenon light (see Figure 1a).
▪ Threshold: intensity of annulus required to completely veil target.

Contrast Enhancement (CE)
▪ 600nm target (100% contrast grating, 5 cpd); 460nm surround (see Figure 1 A(b)).
▪ Threshold: intensity of background required to obscure edge between target and surround.

Photostress Recovery Time (PR)
▪ 570nm target (100% contrast; 5cpd).
▪ 5 second exposure to bleaching photostressor (5° diameter disk; xenon-white light of log 2.5 µW/cm2 intensity).
▪ Threshold: time to recover visibility of target.

### Method: Dynamic Visual Performance

Coincidence Anticipation Timing (CAT)
▪ Task: button press to indicate when a light bar (traveling along 120 LED linear track) reached a specified point.
▪ Velocity randomly varied between 5, 10, 15, and 20 MPH (15 trials for each speed,
   randomly presented; inter-trial intervals randomly varied between 1000-3000 ms). Fixed and Variable Position Reaction Time (FRT/VRT)
▪ Task: button press in response to LED repeatedly presented at same position (FRT) or random location (VFT) along the 120 LED linear track.
▪ 60 trials; inter-trial intervals varied between 1000-3000ms

Temporal Contrast Sensitivity Function (TCSF)
▪ Free-view presentation (through an artificial pupil; see Figure 3A(a)) of 1°, 660nm target centered within 10°, 660nm surround (see Figure 3A(b)).
▪ Threshold: depth of modulation required to detect movement of target presented at frequencies of 1.5, 1.4, 1.0, and 0.4 log Hz.

Critical Flicker Fusion Thresholds (CFF)
▪ Threshold: frequency (Hz) at which the 1°, 660nm target presented at 100% modulation appeared to fuse.

### Method: Macular Pigment Optical Density

Heterochromatic Flicker Photometry
▪ Customized HFP [3] - Macular Metrics Corp., Providence, RI,

### Results

Athletes vs. Non-athletes
▪ Average MPOD at 30' eccentricity: 0.50.
▪ Average age: 20 years.

**Table 1. Static visual performance of athletes (no significant difference from non-athletes).**

| **GL** | **CE** | **PR** |
|---|---|---|
| (log energy) | (log energy) | (seconds) |
| 0.84 | 0.72 | 20.8 |

**Table 2. Dynamic visual performance of athletes (*significantly different from non-athletes, p < 0.05).**

| **Velocity** | **CAT** | **FRT** | **VRT** |
|---|---|---|---|
| 5 mph | 100% | (ms) | (ms) |
| 10 mph | 98% | 217.44 | 225.52 |
| 15 mph | 88% | No significant differences in TCSF or CFF measures | No significant differences in TCSF or CFF measures |
| 20 mph | 62% | No significant differences in TCSF or CFF measures | No significant differences in TCSF or CFF measures |

Zeaxanthin Supplementation
▪ 20mg/d supplementation ongoing.
▪ Preliminary data indicate increases in MPOD and improvement in both static and dynamic visual performance (see Figure 4A for sample data from a subject who completed the 12 week intervention, MPOD change from 0.70 to 0.77).

### Conclusions

Supplementation to increase MPOD may be especially beneficial for baseball players, given the tasks performed and outdoor lighting conditions.

### Example 3

### Macular Pigment: Relations to Fixed and Variable Reaction Time and Coincidence Anticipation Across the Lifespan

### Introduction

High macular pigment optical density (MPOD) relates to improved critical flicker fusion thresholds [1] and temporal contrast sensitivity [2]. Whether improved ability to detect flicker translates to functional changes such as improved reaction time (RT) and coincidence anticipation timing (CAT) is unknown. Three studies were conducted to determine these relations in individuals across the lifespan.
*Study 1:* MPOD & Standard Judgment RT
*Study 2:* MPOD & CAT
*Study 3:* MPOD & Fixed/Variable RT (FRT/VRT)

Subjects:
*Study 1:* N = 49; Mean Age = 54.76 ± 11.97 years
*Studies 2 and* 3: N = 62; Mean Age = 20.71 ± 2.80 years

### Macular Pigment Optical Density:

MPOD was assessed with customized heterochromatic flicker photometry at 30 minutes retinal eccentricity.

### Reaction Time Paradigm:

A standard judgment RT paradigm was used, in which subjects responded with a key press that corresponded to the location of a randomly presented, computerized target appearing in one of four screen quadrants.

### Timing Device (CAT, FRT, VRT):

A novel device was constructed for the CAT and FRT/VRT tasks based on studies using the Bassin Anticipation Timer [3]. The linear track consisted of 120 LEDs specaed 2.02 cm apart along a 10.07 foot linear track. The device utilized a custom-made software program.

### Method

### Coincidence Anticipation Timing:

Individual LEDs along the linear 120 LED track were lit in sequence. This created the appearance of a small, moving light bar. *See* Figure 1B. Subjects were asked to press a button to stop the light bar at a specified point along the track. Bar speed was randomly varied between 5, 10, 15, and 20 MPH across 60 trials. Inter-trial intervals were varied between 1000 ms and 3000 ms.

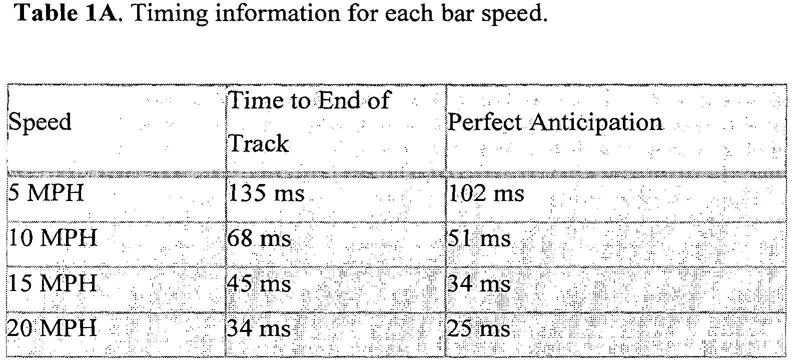

### Fixed and Variable Position Reaction Time:

The fixed task required a button press in response to one of the LEDs, repeatedly presented at the same position on the track. The varied task required a button press in response to one of the LEDS presented at a random location along the 120 LED track. *See* Figure 2B(a), Figure 2B(b),

### Results

### Study 1: MPOD & Standard Judgment RT

MPOD was significantly related to decreased RT (*r* = -0.25, *p* < 0.05). *See* Figure 3B

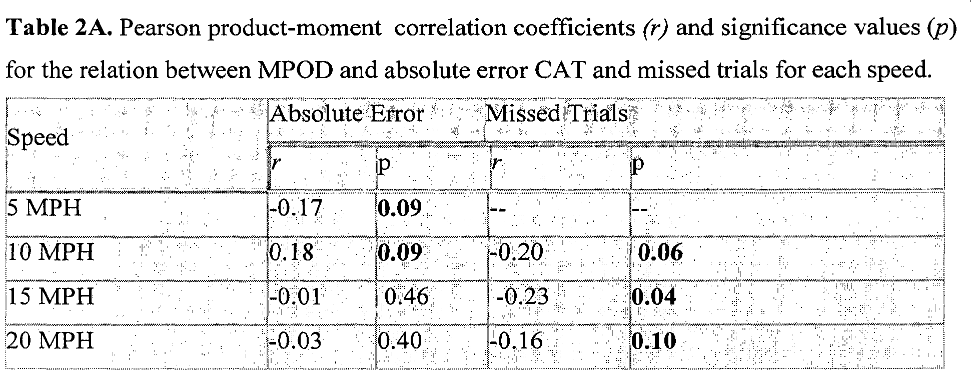

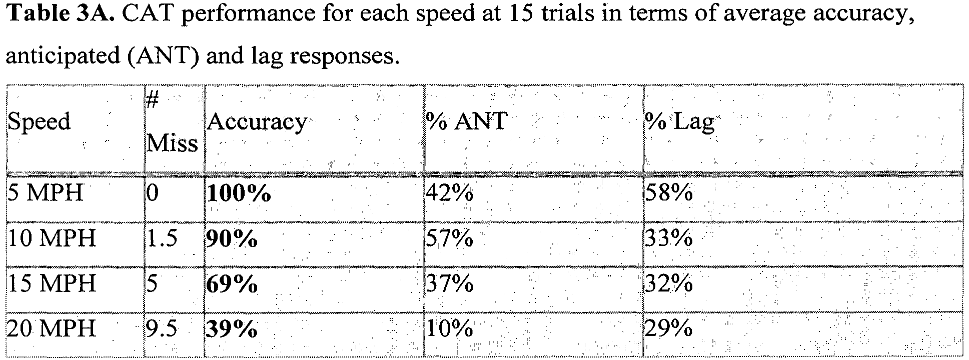

### Study 3: MPOD and Fixed/Variable Position RT:

MPOD was significantly related to FRT (*r* = -0.21, p ≤ 0.05) and VRT (*r* = -0.22, p ≤ 0.05).

### Conclusions

### MPOD and Reaction Time:

MPOD is significantly related to reaction time in middle-age and older adults (Study 1) and in college-aged adults (Study 3). MPOD is significantly related to reaction time as assessed via multiple methods, designed to capture earlier and later stages of visual processing. Study 1: Judgment paradigm, later visual processing; participant must not only see the stimulus, but also make a judgment about where the stimulus is located in space. Study 3: Fixed and variable simple reaction time, early visual processing; participant simply presses a button when the stimulus appears. No judgment necessary.

These results lend support to the neural efficiency hypothesis of macular pigment function [2].

### Coincidence Anticipation:

MPOD was significantly related or trending toward significance to absolute error ion low speed trials, and to number of trials missed on high speed trials. These relationships are complex. MP likely accounts for a small proportion of variance in CAT performance, but CAT performance is likely subject to practice.

### References for Example 3

**[1]** Hammond, BR & Wooten BR. (2005). Ophthal Physiol Opt, 25, 315-319.
**[2]** Renzi, LM & Hammond BR (2010). Ophthal Physiol Opt, 30, 351-357.
**[3]** Millslagle, D.G. (2000). Perceptual & Motor Skills, 90, 498-504.

### Example 4

### Macular Pigment and Visual Motor Function in Young, Healthy Adults

Our experimental results presented below suggest that one result of improved neural efficiency (present in those with higher MP optical density, MPOD) is increased visuomotor performance, including improved fixed (Figure 1C) and variable (Figure 2C) reaction times and increased accuracy in the ability to anticipate and coordinate a motor action with a rapidly appearing visual stimulus (Figure 3C). These pilot data were collected in a cross-section of young, healthy adults, who were not actively taking a Z supplement. Additional pilot data from our laboratory suggests that MPOD relates to balance time (Figure 4C), as well as reaction time using a judgment paradigm (Figure 5C) in healthy, supplement naive elders.

Experiments are conducted to expand upon this pilot work, collected over a number of years. Improving MPOD via Z supplementation is shown to improve neural efficiency and, consequently, visual motor ("visuomotor") performance, in young, healthy adults.

### Methods

### Participants

A total of 60 young, healthy adults, aged 18-30 years, are recruited.

Inclusion and Exclusion Criteria
- Must not have taken lutein or Z supplements in the last 6-months
- Must have best corrected visual acuity (Snellen notation) of 20:40 or better

### Specific Methods

### Macular pigment optical density (MPOD):

MPOD will be measured psychophysically via customized heterochromatic flicker photometry (Stringham 2008). A spatial profile highlighting the fovea (loci at 7.5-minutes, 15-minutes, 30-minutes and 90-minutes, using targets of 15-minutes, 30-minutes, 1-degree and 1.75-degrees in diameter, respectively) will be collected at baseline and at the conclusion of the supplementation period, using a 7-degree parafoveal reference.

### Temporal Contrast Sensitivity Function (tCSF):

The tCSF will be measured using a custom made desktop device (Wooten, Renzi et al. 2010). The depth of modulation necessary to enable flicker detection will be measured at 1.5, 1.4, 1.3, 1.2, 1.1, 1, 0.8, 0.6, and 0.4 log Hz, following procedures outlined by Renzi and Hammond (2010).

### Fixed and Variable Reaction Time (FRT and VRT) and Coincidence Anticipation Timing (CAT):

FRT, VRT, and CAT will be determined using a custom made, wall-mounted, linear light array (see Figure 6C for a schematic). The array consists of 120 LEDs, spaced equally at approximately 1.3 cm apart on a 3.05 meter track. The researcher can isolate and illuminate a single LED at a time in the array (for FRT and VRT testing), or the speed at which the LEDs are illuminated in a sequence, which creates the percept of a rapidly moving light bar (CAT testing).
- *FRT.* For FRT testing, participants will be asked to stand at a distance of 2.5 meters from the linear light array, so that the entire array is easily visible. The researcher will select a specific LED on the light array to use as a test stimulus. The LED chosen will not vary across participants and will be given a white surround on the linear light array, in order to help participants maintain fixation on the correct LED. Participants will be asked to press a button on a keypad as soon as the pre-determined LED is illuminated. The interval between trials will be varied randomly between 1000 and 3000 ms, for 60 total trials.
- *VRT.* For VRT testing, the same procedure and apparatus as FRT testing will be used, with o ne procedural variation: instead of repeatedly illuminating a single, predetermined LED for each trial, any LED within the entire linear light array may be chosen, and the same LED is not illuminated each trial. Instead, a different LED will be randomly chosen for illumination from trial to trial
- *CAT testing.* In order to measure CAT, the same linear light array described previously will be used. LEDs will be illuminated and then turned off rapidly in sequence, which creates the percept of a rapidly moving light bar. Both the amount of time between trials will be varied (between 1000 and 3000 msec), as well as the velocity of the light bar. Participants will be asked to make a button press coincide with the arrival of the moving light bar to a specific, pre-determined LED on the array, positioned at 2.29 meters on the 3.05 meter track. The specific LED will be given a white surround on the linear light array, in order to help participants keep the ending spot within sight while tracking the light bar. The light bar's velocity will be randomly varied between 5 mph (1.02 sec between onset and the button press for perfect anticipation), 10 mph (0.51 sec between onset and the button press for perfect anticipation), 15 mph (0.34 sec between onset and the button press for perfect anticipation), and 20 mph (0.25 sec between onset and the button press for perfect anticipation) between trials. A total of 60 trials will be completed.

### Design

The general design of this study is a randomized, double-masked, placebo-controlled trial. Participants will be randomized into two groups: the supplement group (n = 50) and the placebo group (n = 10). The supplement group will be divided into 2 groups. One group (n=25) will be supplemented with 20 mg of zeaxanthin per day. The second group (n=25) will be supplemented by a formula (to be provided by ZeaVision) that will contain a mixture of zeaxanthin, lutein and Omega-3s. Randomization will be conducted by the researcher overseeing the trial, who will be the only member of the research team who knows supplement status (placebo vs. active supplement). Consequently, the researcher will not be actively collecting data on these participants. The rest of the research team will be masked to supplementation status. When the analyses have been conducted and the data are unmasked, participants in the placebo group will be given a four month's supply of Z supplements and will be encouraged to return to the Human Biofactors and Vision Sciences Laboratories for free follow-up testing should they wish to see if active supplementation is benefitting them.

### References for Example 4

Renzi, L. M. and B. R. Hammond, Jr. (2010). "The relation between the macular carotenoids, lutein and zeaxanthin, and temporal vision." Ophthalmic & Physiological Optics: The Journal Of The British College Of Ophthalmic Opticians (Optometrists) 30(4): 351-357.
Stringham, J. M., Hammond, B.R., Nolan, J.M., Wooten, B.R., Mammen, A., Smollon, W., Snodderly, D.M. (2008). "The utility of using customized heterochromatic flicker photometry (cHFP) to measure macular pigment in patients with age-related macular degeneration." Experimental Eye Research 87: 445-453.
Wooten, B. R., L. M. Renzi, et al. (2010). "A practical method of measuring the human temporal contrast sensitivity function." Biomed Opt Express 1(1): 47-58.

### References for Background of the Invention

1. Snodderly DM, Auran JD, Delori FC: The macular pigment. II. Spatial distribution in primate retinas. Investigative Ophthalmology & Visual Science 1984, 25(6):674-685.
2. Craft NE, Haitema, T.B., Garnett, K.M., Fitch, K.A., Dorey, C.K.: Carotenoid, tocopherol, and retinol concentrations in elderly human brain. J Nutr Health Aging 2004, 8(3):156-162.
3. Wooten BR, Hammond, B.R., Land, R.I., Snodderly, D.M.: A practical method for measuring macular pigment optical density. Investigative Ophthalmology and Visual Science 1999, 40:2481-2489.
4. Renzi LM, Hammond BR, Jr.: The relation between the macular carotenoids, lutein and zeaxanthin, and temporal vision. Ophthalmic & Physiological Optics: The Journal Of The British College Of Ophthalmic Opticians (Optometrists) 2010, 30(4):351-357.

## Claims

1. Non-therapeutic use of Zeaxanthin (Z) in a method of improving reaction time and coincidence anticipation ability in a subject in need, the method comprising administering to the subject a pharmaceutically effective amount of zeaxanthin (Z),
wherein the subject is required to perceive and react to temporally varying stimuli under lighting conditions that are known to be detrimental to visual function,
wherein the subject does not suffer from an ocular disorder.

2. Non-therapeutic use of Zeaxanthin (Z) according to claim 1, wherein the zeaxanthin (Z) is in substantially pure enantiomeric form.

3. Non-therapeutic use of Zeaxanthin (Z) according to any of claims 1 or 2, wherein improved reaction time and coincidence anticipation ability is **characterized by** enhanced static and dynamic visual performance.

4. Non-therapeutic use of Zeaxanthin (Z) according to any of claims 1-3, wherein the zeaxanthin (Z) is administered to the subject before the subject encounters glare conditions.

5. Non-therapeutic use of Zeaxanthin (Z) according to any of claims 1-4, further comprising co-administering a fatty acid to the subject.

6. Non-therapeutic use of Zeaxanthin (Z) according to any of claims 1-5, wherein the fatty acid is selected from the group consisting of an omega 3, omega 6 and an omega 9 fatty acid.

## Patentansprüche

1. Nichttherapeutische Verwendung von Zeaxanthin (Z) bei einem Verfahren zum Verbessern von Reaktionszeit und Koinzidenzantipizationsfähigkeit bei einem bedürftigen Probanden, wobei das Verfahren ein Verabreichen einer pharmazeutisch wirksame Menge an Zeaxanthin (Z) an den Probanden umfasst,
wobei der Proband variierende Stimuli unter Beleuchtungsbedingungen, von denen bekannt ist, dass sie nachteilig für die Sehfunktion sind, wahrnehmen und darauf reagieren muss,
wobei der Proband nicht unter einer Augenstörung leidet.

2. Nichttherapeutische Verwendung von Zeaxanthin (Z) nach Anspruch 1, wobei das Zeaxanthin (Z) im Wesentlichen in reiner enantiomerer Form vorliegt.

3. Nichttherapeutische Verwendung von Zeaxanthin (Z) nach einem beliebigen der Ansprüche 1 oder 2, wobei verbesserte Reaktionszeit und Koinzidenzantipizationsfähigkeit **gekennzeichnet sind durch** eine erhöhte statische und dynamische Sehleistung.

4. Nichttherapeutische Verwendung von Zeaxanthin (Z) nach einem beliebigen der Ansprüche 1 bis 3, wobei das Zeaxanthin (Z) dem Probanden verabreicht wird, bevor der Proband einer Blendung ausgesetzt wird.

5. Nichttherapeutische Verwendung von Zeaxanthin (Z) nach einem beliebigen der Ansprüche 1 bis 4, weiter umfassend eine gemeinsame Verabreichung einer Fettsäure an den Probanden.

6. Nichttherapeutische Verwendung von Zeaxanthin (Z) nach einem beliebigen der Ansprüche 1 bis 5, wobei die Fettsäure gewählt ist aus der Gruppe, bestehend aus einer Omega-3-, einer Omega-6- und einer Omega-9-Fettsäure.

## Revendications

1. Utilisation non thérapeutique de zéaxanthine (Z) dans un procédé pour améliorer le temps de réaction et la capacité d'anticipation des coïncidences chez un sujet dans le besoin, le procédé comprenant l'administration, au sujet, d'une quantité pharmaceutiquement efficace de zéaxanthine (Z),
dans laquelle il est demandé au sujet de percevoir et de réagir à des stimuli variables dans le temps dans des conditions d'éclairage qui sont connues pour être nuisibles à la fonction visuelle,
dans laquelle le sujet ne souffre pas d'un trouble oculaire.

2. Utilisation non thérapeutique de zéaxanthine (Z) selon la revendication 1, dans laquelle la zéaxanthine (Z) est sous une forme énantiomérique sensiblement pure.

3. Utilisation non thérapeutique de zéaxanthine (Z) selon l'une quelconque des revendications 1 et 2, dans laquelle un temps de réaction et une capacité d'anticipation des coïncidences améliorés sont **caractérisés par** une performance visuelle statique et dynamique améliorée.

4. Utilisation non thérapeutique de zéaxanthine (Z) selon l'une quelconque des revendications 1-3, dans laquelle la zéaxanthine (Z) est administrée au sujet avant que le sujet ne rencontre des conditions d'éblouissement.

5. Utilisation non thérapeutique de zéaxanthine (Z) selon l'une quelconque des revendications 1-4, comprenant en outre l'administration conjointe d'un acide gras au sujet.

6. Utilisation non thérapeutique de zéaxanthine (Z) selon l'une quelconque des revendications 1-5, dans laquelle l'acide gras est sélectionné dans le groupe constitué par un acide gras oméga 3, un acide gras oméga 6 et un acide gras oméga 9.
